# EUROPEAN PATENT APPLICATION

(11) **EP 3 805 402 A1**
(43) Date of publication of application: **14.04.2021**
(21) Application number: 19202465.1
(22) Date of filing: 10.10.2019
(51) Int. Cl.: C12Q 1/34, G01N 33/543, G01N 33/58, G01R 33/02, G01R 33/12

(54) **NON-INVASIVE METHOD FOR DETECTION OF ENZYME ACTIVITY IN VIVO, SUBSTRATES AND A DEVICE THEREFORE**

(71) Applicant: ETH Zurich, 8092 Zurich (CH)
(72) Inventor: SCHÜRLE, Simone, Zürich 8006 (CH); CHRISTIANSEN, Michael, 8037 Zürich (CH); RISTANOVIC, Dragana, 8600 Dübendorf (CH)

(57) **Abstract**

The invention relates to methods for the detection of enzymatic activity, in particular, to in vivo methods. It relates to a non-invasive method for in vivo enzyme activity detection, in particular, activity of proteinases, to substrates specifically developed for these methods and to a device detecting product formation of the enzyme to be tested based on determination of signals produced by the substrates and/or its products.

## Description

The invention relates to methods for the detection of enzymatic activity, in particular to *in vivo* methods. It relates to a non-invasive method for *in vivo* enzyme activity detection, in particular activity of proteinases, to substrates specifically developed for these methods and to a device detecting product formation of the enzyme to be tested based on determination of signals produced by the substrates and/or its products.

### Background of the Invention

Enzymes are macromolecular biological catalysts that accelerate chemical reactions, such as metabolic processes in a cell. The enzymes act upon molecules called substrates. These substrates are converted into products. Most enzymes are proteins, although a few are catalytic RNA molecules (ribozymes), having a defined three-dimensional structure being responsible for specificity of the enzyme.

Enzyme activity can be affected by other molecules: inhibitors are molecules that decrease enzyme activity, and activators are molecules that increase activity. Some enzymes need non-protein molecules to show full activity, called cofactors. Preferred enzymes in regard to the present invention are hydrolases, which are classified as EC 3 in the EC number classification of enzymes. Hydrolase is a class of enzyme that commonly perform as biochemical catalysts that use water to break a chemical bond, which typically results in dividing a larger molecule to smaller molecules.

Nowadays, enzymes are recognized as exceptionally important molecules that are engaged in numerous vital life processes. Because certain states of enzyme activity can affect, lead to or signal specific diseases, it is not surprising that enzymes have been actively investigated not only as potential therapeutic targets but also as biomarkers. In particular, certain types of proteases can be used as biomarkers and/or prognostic markers, therefore different types of diagnostics measuring proteases' activities have been developed. Several research articles introduce new methods or systems targeting specific enzymes, in cells and in clinical samples such as blood or excrements. One goal, of recent technological developments is detecting and imaging enzyme and in particular, protease activities in living organisms using different imaging modalities, such as fluorescence and bioluminescence resonance energy transfer systems. These promising enzyme activity sensors are expected to identify and contribute to the understanding of the specific role of enzymes in particular, biological processes and have great potential as high throughput drug screening tools for screening novel inhibitors as therapeutic drugs. Most of these techniques are not compatible with measuring the activity of proteases in living organisms, at least not at human scale. Although magnetic resonance (MR)-based imaging techniques are scalable, since the analysis requires a bulky MR scanner, this method is not suitable for routine diagnostic use of enzyme activity in vivo. Therefore, existing techniques do not allow in situ detection and monitoring of enzyme activities related to a large range of diseases. Proteases play a critical role in many physiological and pathological processes such as protein catabolism, blood coagulation, cell growth and migration, tissue arrangement, morphogenesis in development, activation of zymogens, release of hormones and pharmacologically active peptides from precursor proteins, and transport of secretory proteins across membranes. Diseases associated with and/or caused by an altered protease activity comprise for example inflammatory diseases such as arthritis, autoimmune diseases, bacterial or viral infections, cancer and metastasis, impaired blood coagulation, cardiovascular diseases, lung diseases such as COPD or asthma, and metabolic diseases.

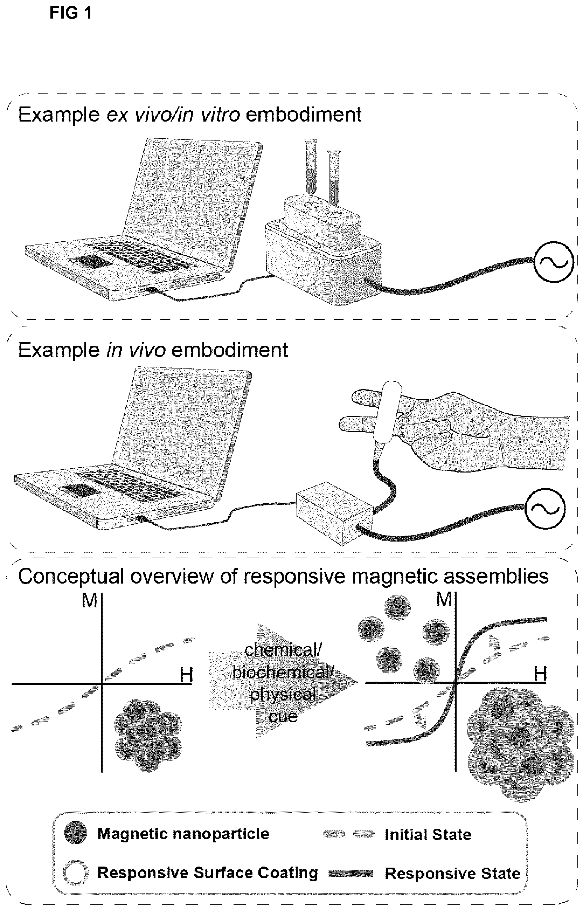

The present invention relates to methods for the detection of enzymatic activity, in particular, to in vivo methods using either responsive and magnetic nanoparticles or responsive and acoustically detectable microbubbles to monitor enzyme activity within a living body. The invention refers further to responsive magnetic nanoparticles and an accompanying device that detects them via magnetic induction. The inductively detected signal from these responsive magnetic nanoparticles which may form nanoassemblies can then be used to make inferences about the chemical, biochemical, or physical environment they experience (Figure 1). In one embodiment of the invention, responsive magnetic nanoassemblies would be held together by selectively cleavable enzyme substrates, e.g. peptides, such that their disassembly, registered as an inductively detected change in their magnetization vs field response, would indicate the activity of a specific enzyme, e.g. a proteolytic enzyme. The non-invasiveness and high penetration depth of magnetic fields allow embodiments of the invention to be employed either directly in patients (in vivo) or on samples collected from patients (ex vivo) for diagnostic purposes. Other embodiments of the invention include equipment for analysis of chemical, biochemical, or physical conditions in vitro or in non-biological systems.

### DESCRIPTION OF THE INVENTION

It is therefore an object of the invention to provide a method for detection of enzymatic activity in vivo comprising the following steps:
a) incorporation of substrates being selectively cleavable by the enzyme of interest, and
b) non-invasive read-out of enzymatic activity.

It is preferred that the substrates are part of a responsive surface coating of magnetic nanoparticles or microbubbles. It is further preferred that the substrate is specific for an enzyme of interest. The term "enzyme" as used herein refers to any protein or conjugated proteins produced by living organisms and functioning as biochemical catalysts thus, being capable of catalyzing a chemical reaction. Preferred enzymes in regard to the present invention are hydrolases. Preferred hydrolases are esterases, such as nucleases, phosphodiesterases, lipases, and phosphatases, glycoysl hydrolases (glycosidases), and proteases, respectively peptidases. Especially preferred are hydrolases acting on peptide bonds (peptidases/proteases). A protease or proteolytic enzyme (commonly also called a peptidase or proteinase, wherein these terms are historically used with slightly different meanings, but today are used mostly idiomatically) is an enzyme that catalyzes (increases the rate of) proteolysis, the breakdown of proteins into smaller polypeptides or single amino acids. They do this by cleaving the peptide bonds within proteins by hydrolysis, a reaction where water breaks bonds. Proteases are involved in many biological functions, including digestion of eaten proteins, protein catabolism (breakdown of old proteins), and cell signalling.

One of the properties of enzymes that makes them important as diagnostic and research tools is the specificity they exhibit relative to the reactions they catalyze. This means enzymes show a selectivity to their substrate. The specificity is the ability of an enzyme to choose exact substrate from a group of similar chemical molecules. Specificity arises by virtue of the three-dimensional structure of the site at which the enzyme binds with the target substrate. In this way, enzymes only bond with a given range of chemical reactions. Some enzymes include a secondary structure that fits the chemical substrate and may function as a proofreader, which further ensures enzyme specificity. Some enzymes that catalyze a variety of chemical substrates perform differently depending on the substrate with which they bond. For example, when an enzyme bonds with a preferred substrate, the enzyme may conform to the substrate more completely than it would if it bonded with a non-preferred substrate.

Hence, a few enzymes exhibit absolute specificity; that is, they will catalyze only one particular reaction. Other enzymes will be specific for a particular type of chemical bond or functional group. In general, there are four distinct types of specificity:
- Absolute specificity - the enzyme will catalyze only one reaction.
- Group specificity - the enzyme will act only on molecules that have specific functional groups, such as amino, phosphate and methyl groups.
- Linkage specificity - the enzyme will act on a particular type of chemical bond regardless of the rest of the molecular structure.
- Stereochemical specificity - the enzyme will act on a particular steric or optical isomer.

Step a) of the method according to the invention refers to an incorporation of substrates being selectively cleavable by the enzyme of interest. These substrates may be conjugated to nanoparticles which alter at least one physical or physicochemical characteristic depending on the substrate or respectively the enzyme activity. More particular, the physical or physicochemical characteristic of the nanoparticle alters in case the substrate is cleaved by the enzyme of interest. In addition, it is preferred that the alteration of the physical or physicochemical characteristic of the nanoparticle can be determined "in situ" without the use of an invasive method. Step a) of the present invention may comprise background measurements made before the incorporation of the substrates.

The term "in situ" as used herein describes the way a measurement is taken, that is, in the same place the enzyme activity is occurring without isolating the enzyme from a patient's body also not by taking a sample (such as a tissue sample, blood, urine or the like). The term "non-invasive" means that determination of enzyme activity does not involve any surgical technique. Hence, no tissue of the patient's body is destroyed or injured.

Consequently, the present invention refers further to a method, wherein step b) comprises determination of enzyme activity (locally) within a human or animal body.

The incorporation of substrates or respectively the nanoparticles comprising the substrates may comprise administration of the substrates/nanoparticles. This administration can be oral (e.g. in form of a suspension) or intravenous. Possible alternative routes of administration include the transdermal or transmucosal routes, epidural, nasal, intramuscular, intra-arterial, and intravitral.

The present invention refers further to a method according the invention, wherein the substrate is selected from the group consisting of polypeptides, including those that incorporate unnatural amino acids, sugars, polynucleotides, lipid, glycosaminoglycan and conjugates thereof.

One detection principle suitable for the methods of the present invention and in particular for non-invasive read-out of enzymatic activity is determining the reaction of magnetic particles to a magnetic field and in particular their altered or altering reaction to a magnetic field depending on the enzyme activity on the substrate comprised by the magnetic particles.

Thus, the present invention provides further methods for detection of enzyme activity comprising the following steps:
a) incorporation of substrates being selectively cleavable by the enzyme of interest, and
b) non-invasive determination of the reaction of magnetic nanoparticles comprising the substrate to a magnetic force, wherein the reaction depends on enzyme activity altering (cleaving) the substrate. It is preferred that the reaction of the magnetic nanoparticles determined is "inductive detection".

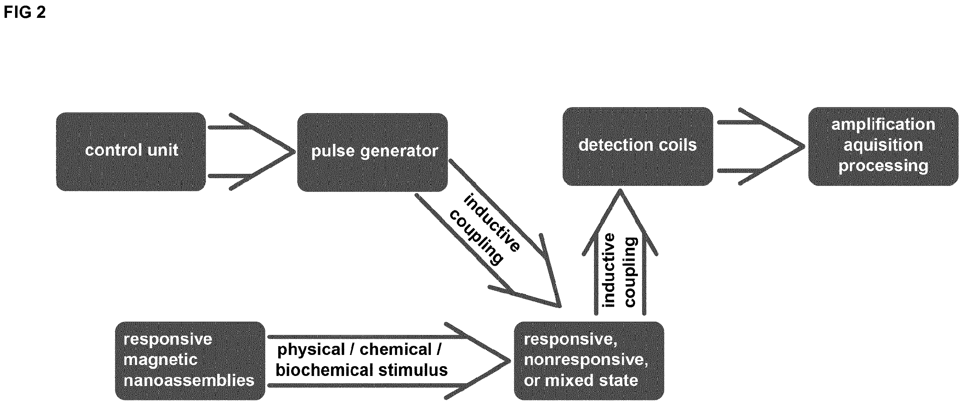

Those of skill in the art will understand "inductive detection" of magnetic nanoparticles to entail the coupling of time changing magnetization to induced voltages in circuits designed to amplify and detect them. Determination of inductive detection is possible using a device consisting of a pulsed field magnetometer that detects responsive magnetic nanoassemblies (see Figure 2).

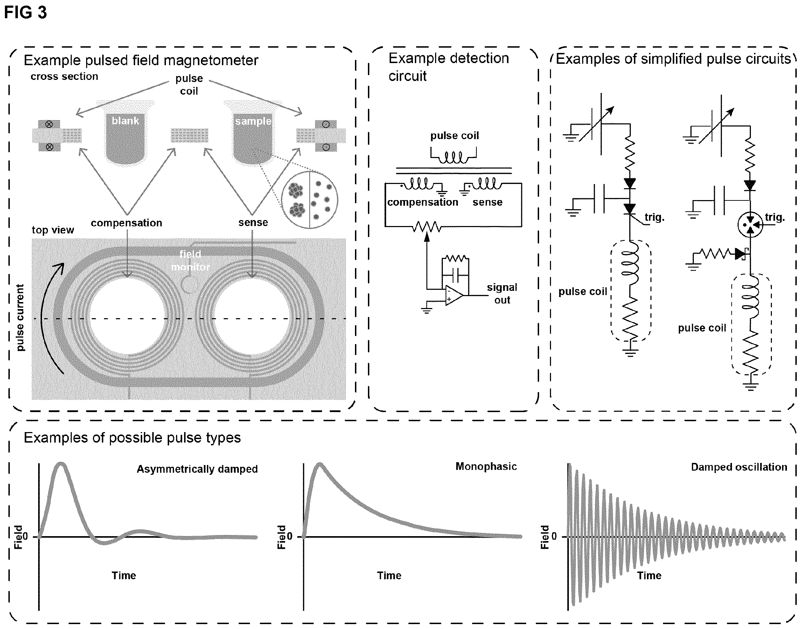

The present invention refers to a magnetometer adapted to perform the method of the invention. In one embodiment, the pulsed field magnetometer includes a pulse coil with a drive circuit that generates a moderate to high amplitude magnetic field (from mT to T) for short durations (from ns to µs). Thereby it is preferred that the changing magnetic field changes its amplitude from a range of 0 T to 1T within a period of 1ns to 10 µs. This rapidly changing magnetic field induces a voltage in a set of detection coils, which are inductively coupled to a sample, such as fluid in a vial placed in the system, and can be designed to isolate the voltage signal arising from its changing magnetization. The signal can be amplified, recorded, and analyzed. In one embodiment, the pulse coil may consist of electrically conductive spirals resting on either side of a printed circuit board or planar geometry on which conductive features can be patterned with high resolution and geometric symmetry (Figure 3). In another embodiment, the detection coil is incorporated as traces on the printed circuit board surrounding holes dimensioned to incorporate samples (e.g. a vial to which magnetic nanoassemblies have been added or e.g. finger joints in which magnetic nanoassemblies had been injected.) In such an embodiment, the detection coil can consist of two parts: a sense coil that is inductively coupled to the sample and the driving field and a compensation coil. The compensation coil may serve to compensate for the induced voltages arising in the sense coil from sources other than the changing magnetization of the sample, particularly the pulsed field applied to the sample, as well as and linearly susceptible background contributions from a blank sample placed in the compensation coil.

Although those of skill in the art will understand a "pulse" to refer to a magnetic field applied for a limited duration, the particulars of the time dependence of this pulse (e.g. biphasic, monophasic, half sine, full sine, decaying oscillation, etc.) may vary. In some in vivo embodiments, the time scale of the pulsed magnetic field produced by the pulse circuit would be designed to be sufficiently short to avoid dissipation of significant energy in the tissue or nerve stimulation of the kind experienced with the pulsed fields in transcranial magnetic stimulation.

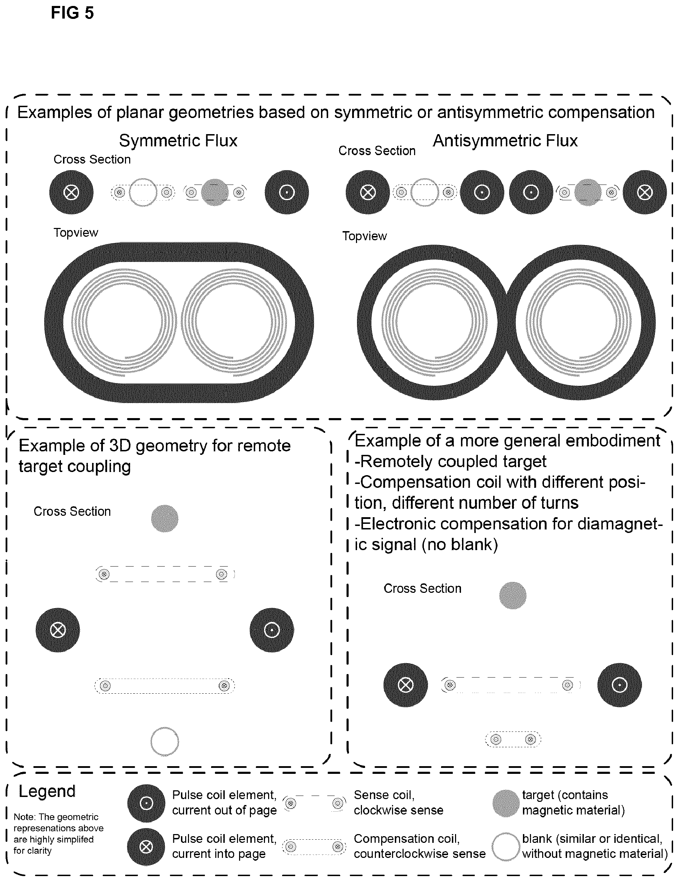

Another embodiment of the invention refers to a magnetometer, suitable to impose a magnetostatic gating field by permanent magnets or electromagnets to produce spatially selective measurement of magnetic material. Further embodiments of the magnetometer are arranged in a way that the pulse coil and detection coils are enlarged such that it encompasses human scale targets of physiological interest, including but not limited to joints and the abdomen. This results in an increased penetration depth which may be important to measure a signal within deep regions of a body. Numerous other arrangements of the sense and compensation coils are possible (see for example Figure 5). The background subtraction principle of the compensation coil can be carried out with spatially symmetric or antisymmetric fields, or incomplete cancellation compensated by data acquisition circuits and signal processing. Geometries could be altered to reduce inductance or provide more spatially focused magnetic coupling.

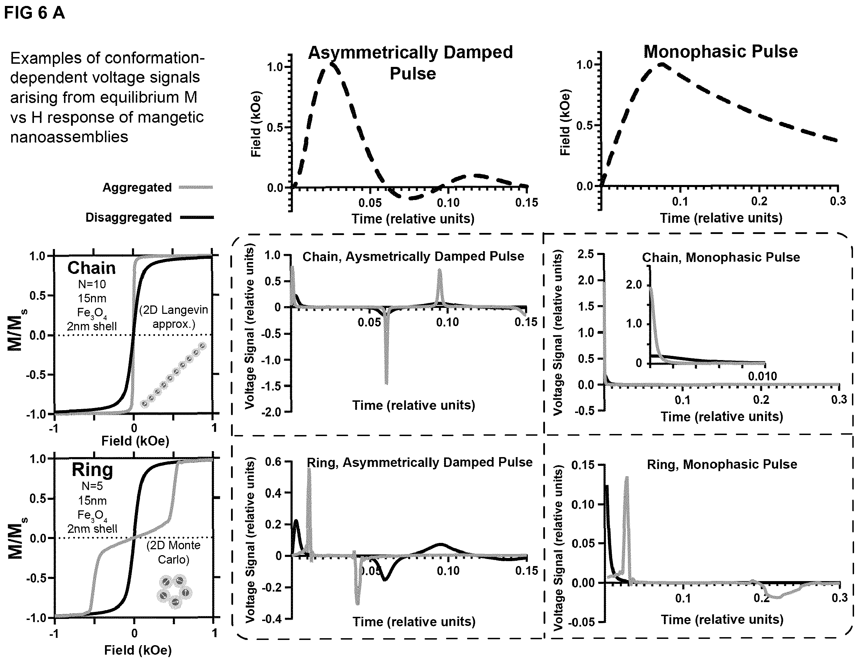

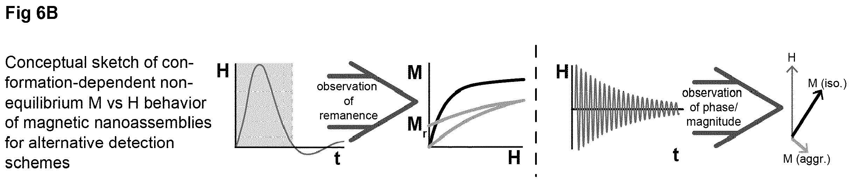

In a preferred embodiment of the inventive method, signal processing involves reconstructing an M vs H curve from the detected voltage signal. The curves may be normalized to their saturation values and low field susceptibility can be determined. In another embodiment, rapid pulses result in observable differences in the magnetization curve during the rising and falling times of the field in a way that depends on the aggregation state of the magnetic nanoparticles, allowing e.g. for the observation of aggregation dependent magnetic remanence. Alternatively, low amplitude decaying sinusoidal pulses could be applied at two or more frequencies to observe changes in the timescale of magnetization reversal. The basic concepts for these strategies of measurement of differences in aggregation of nanoparticles within the present invention are illustrated in Figure 6, along with a simulated voltage signal arising under the noted set of assumptions.

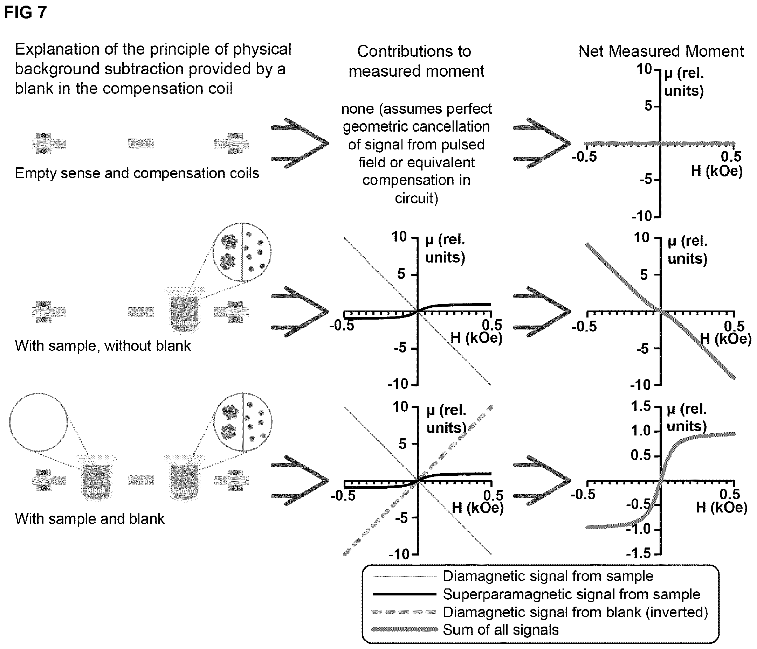

The following possibilities for the refinement of signal acquisition and interpretation (in particular physical background subtraction) may be part of step b) of the method according to the invention, respectively the elements therefore may be part of the magnetometer according to the invention. The diamagnetic contribution to the signal arising from the solution or tissue surrounding the responsive magnetic nanoassemblies can be physically cancelled by the incorporation of e.g. a sample having similar characteristics or corresponding body part (e.g. a finger of the other hand, or the other knee of the patient) in the compensation coil that does not contain magnetic particles (Figure 7). This reduction in physically detected background boosts the detection sensitivity (i.e. reduce the quantity of magnetic material required for signal acquisition). Background measurements could also be made before the introduction of responsive magnetic nanoparticles (step a) of the inventive method) either to adjust adaptive elements in the signal acquisition circuit and/or save a known background signal for digital subtraction. Signal averaging over sequential pulses could be used to reduce noise, and the signal would be amplified, perhaps incorporating magnetic shielding to protect the amplification and signal acquisition circuit from the pulsed magnetic field.

Another aspect of the invention refers to magnetic nanoparticles designed to be used within the inventive method. The magnetic nanoparticles of the invention comprise a substrate for an enzyme of interest and being suitable to form magnetic nanoassemblies wherein the magnetic nanoassemblies produce a detectable signal difference in response to substrate conversion by the enzyme of interest. It is further preferred that the magnetic nanoparticle as well as the nanoassemblies are smaller than 1 µm in diameter, which is below critical size thresholds for intravenous applications.

The invention refers further to magnetic nanoassemblies consisting of magnetic nanoparticles according to the invention. This nanoassembly can be being designed to produce a (e.g. inductively) detectable signal difference capable of distinguishing between states coupled to environmental stimuli, such as disassembly in response to the activity of selected enzymes or to pH conditions. One embodiment of the invention refers to magnetic nanoassemblies, wherein the detectable signal difference is caused by disassembly in response to the activity of the enzyme of interest.

Magnetic dipole interactions between the constituent nanoparticles of a nanoassembly offer a basis for a behaviour in a magnetic field which can be influenced and determined. In the case of magnetization versus field response, interactions can influence overall magnetization by altering the net moment of the nanoassembly, either by reinforcing magnetic ordering (as in geometries with open flux paths, such as chains) or partial cancellation (as in geometries that produce closed flux paths such as rings, clusters, or shells). Simulations of the statistical equilibrium or dynamic behaviour of the magnetic moments of various geometries of nanoassemblies may be used to predict and design cluster architectures that exhibit detectable signal differences between their responsive and non-responsive states. This includes, but is not limited to, Monte Carlo simulations accounting for dipole interactions between particles, intrinsic contributions such as individual particle anisotropy, and the influence of an applied field.

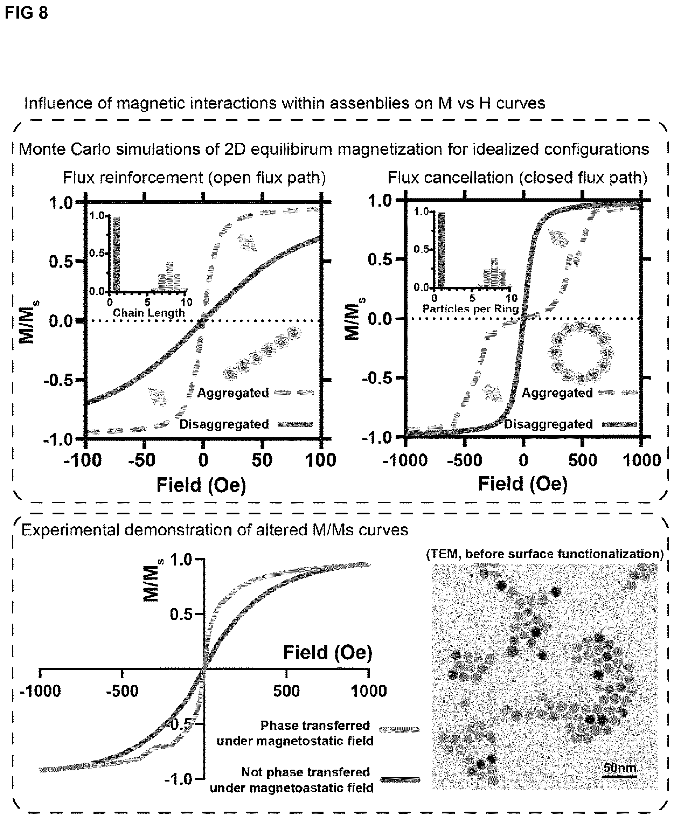

The basic principle of responsive aggregates is shown in the results of 2D Monte Carlo models of equilibrium magnetization versus field curves for chains and rings, depicted in Figure 8. Chains and rings represent idealized cases that demonstrate assemblies in which energetic interactions between particles lead to flux paths reinforcement (chains) or cancelation (rings). More generally, the dynamics of magnetization response can also be influenced, leading to the possibility of measurement of remanence under the influence of a pulsed magnetic field, or phase shift in a decaying periodic burst (see Figure 6). Consequently, the change of the geometry of nanoassemblies can be determined.

Physical parameters or properties of the nanoparticles according to the invention that can be varied to produce different nanoassemblies and to influence their geometry include size, composition, shape, the surface chemistry incorporated into these magnetic nanoparticles (thickness, functional groups, chemical linkages, etc.).

The nanoassemblies of the inventive nanoparticles may have one of the following morphologies: a flexible or rigid chain of nanoparticles, closed chains such as rings, a stochastic cluster, core shell assemblies, wherein the core is formed by the nanoparticles, and assemblies with substructures. Further details on suitable or preferentially used sizes and materials of the nanoparticles as fundamental building block are listed below.

Multiple mechanisms of responsiveness are possible. In one embodiment, the presence of a substance of interest, such as a matrix metalloproteinase in its active form, leads to the cleavage of peptides that hold the responsive magnetic aggregates together. In another embodiment, a physical response of the surface coating, such as swelling in response to deprotonation, could alter the distance between constituent particles, offering a means to detect pH. The essential feature is a change in magnetization response based on disassembly or altered magnetic interaction within the nanoassembly.

Thus, the present invention provides methods for detection of pH changes comprising the following steps:
a) incorporation of magnetic nanoparticles forming nanoassemblies or of magnetic nanoassemblies, and
b) non-invasive determination of a change in magnetization of the nanoassemblies taking place in response to a change of the pH-value of the surrounding tissue. It is possible that the magnetization changes because the nanoassemblies disassemble or change their configuration in response to swelling caused by deprotonation of molecules conjugated to the nanoparticle. The term "conjugated" means that the substrate has been attached to the nanoparticle either by a covalent bond or be mediated by electrostatic interactions such as hydrogen bonds or van der Waals forces between different molecules (different peptide chains).

The present invention provides further methods for detection of enzyme activity comprising the following steps:
a) incorporation of substrates being selectively cleavable by the enzyme of interest and being part of nanoassemblies or nanoparticles forming nanoassemblies (after incorporation), and
b) non-invasive determination of the reaction of the magnetic nanoparticles or magnetic nanoparticles comprising the substrate to a magnetic force, wherein the reaction depends on enzyme activity altering (cleaving) the substrate.

The magnetic nanoparticles of the present invention may consist of a magnetic core with a shell comprising the substrate of the enzyme of interest. The shell can be made by surface chemistry. This surface chemistry or the shell may be engineered to allow for biochemical linkages between single nanoparticles.

The core may comprise or be made of iron, iron sulphite, iron oxide, doped iron oxides (Mn, Co, Zn, Ni), Ni, Co, CoPt alloys or combinations thereof. The diameter of the nanoparticle core can vary be between 1 nm and 50 nm and is preferred between 5 nm and 30 nm.

The surface coating may comprise or consist of polymers, silica or ligands having functional groups to which the substrates can linked using known chemical reactions or to which a molecule may be linked which is able to bind the substrate. For example, amphiphilic surface coatings with polymers such as poly(maleic anhydride-alt-1-octadecene) linked with functionalized PEG or poly(aspartic acid) prepared from polysuccinimide linked with otadecylamine and functionalized PEG sidechains could be used. As an alternative to PEG, other hydrophilic polymers could be substituted, such as polyacrylic acid or polyacrylamide.

The substrate should be part of the linkage or influence the linkage between single nanoparticles causing the formation of the magnetic nanoassemblies. There are different possibilities how the substrate can influence the linkage or participate in the linkage. Therefore, the substrate can be conjugated to a molecule being also comprised by the magnetic nanoparticle. This binding may be the specific or non-specific binding of two proteins, such as a ligand binding to its receptor or respectively antibody-antigen binding, or of two peptides (two binding motifs binding to each other), wherein the activity of the enzyme lowers binding affinity. Alternatively, two binding motifs binding to the same receptor or antibody may be linked by a substrate for the enzyme of interest and the magnetic nanoparticle comprises the respective receptor or antibody. Thus, the substrate may link two nanoparticles by being conjugated to each of them. After cleavage of the substrate this linkage is destroyed. Alternatively, the substrate may be conjugated to one nanoparticle and can further be conjugated to another nanoparticle by binding to a biomolecule being part of that nanoparticle, wherein cleavage of the substrate by the enzyme minimizes binding affinity of the substrate for that biomolecule.

In more general words, the substrate can be conjugated to two different nanoparticles linking them to form nanoassemblies. Therefore, the substrate may be conjugated to at least one molecule within the shell of the magnetic nanoparticle.

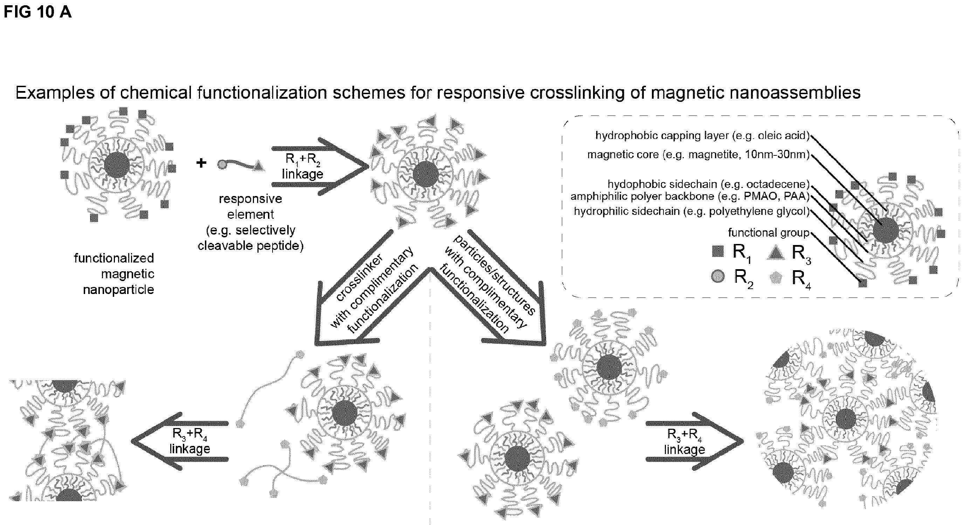

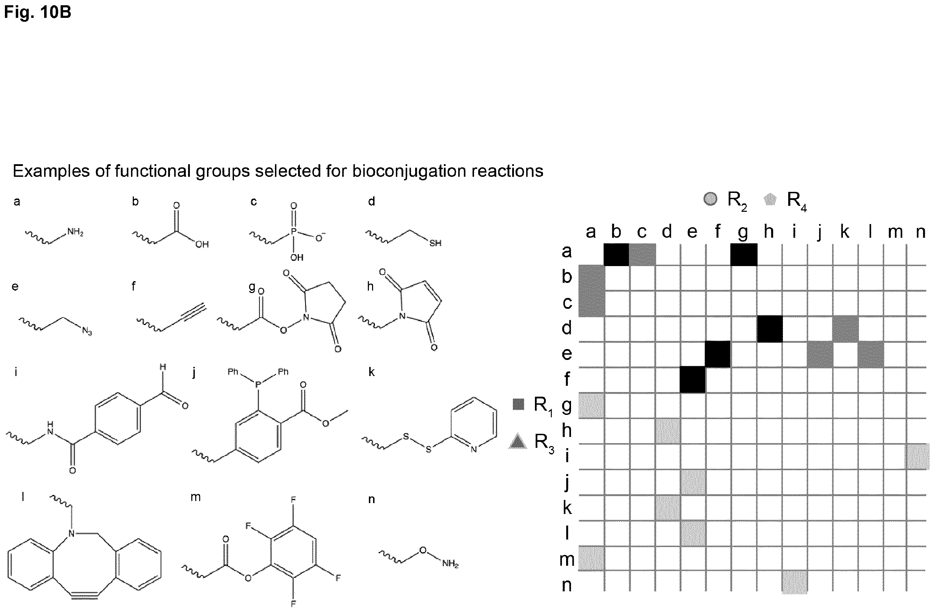

Figure 10 shows a generalized scheme envisioned for the formation of biochemical linkages between particles. In the embodiments with responsiveness arising from selective or preferential cleavage of peptides, the biochemical linkers are selected to incorporate the responsive features. Figure 10 shows a generalized example of the chemistry behind peptide incorporation, though alternative schemes can be anticipated by those of skill in the art.

Possible methods to create aggregates of magnetically interacting nanoparticles include self-assembly, assisted self-assembly (e.g. under the influence of an applied magnetostatic field, which may be constant, pulsed, or rotating, uniform or nonuniform), or templated assembly (e.g. with microfabricated flux paths driven by magnetic interactions or chemical interactions with existing pre-existing structures such as liposomes or planar self-assembled block copolymers).

The magnetic nanoparticles of the present invention may further have the following features: The nanoparticles may be smaller than 1 µm in diameter. The nanoparticles should be suitable to form nanoassemblies, wherein the nanoassemblies are mediated by a substrate for an enzyme of interest and wherein cleavage of the substrate by the enzyme of interest alters their reaction to a magnetic force. The magnetic nanoparticles may have a good tolerability to patients.

The magnetic nanoparticles or magnetic nanoassemblies of the present invention are suitable for use within the methods of the present invention. They are in particular suitable for use in a method of diagnosis in vivo of a disease associated with or caused by an altered activity of an enzyme of interest.

Another detection principle suitable for the methods of the present invention and particular for non-invasive read-out of enzymatic activity is based on microbubble comprising a substrate for an enzyme of interest and wherein the microbubble alters its reflection of ultrasound in response of substrate conversion by the enzyme of interest. Consequently, step b) of the inventive method may be based on an acoustic readout that informs about the chemical, biochemical, or physical environment experienced by chemically encapsulated microbubbles.

Thus, the present invention provides further methods for detection of enzyme activity comprising the following steps:
a) incorporation of substrates being selectively cleavable by the enzyme of interest, and
b) non-invasive determination of the change of ultrasound reflection by microbubbles comprising the substrate, wherein the change in reflection depends on enzyme activity altering (cleaving) the substrate.

Another aspect of the invention are microbubbles suitable to be used within the method of the present invention. Therefore, the microbubbles may be suitable as an ultrasound contrast agent showing enzyme activity. The microbubbles according to the invention can comprise a (cleavage) substrates for an enzyme of interest.
In addition, targeting ligands that bind to receptors characteristic of a specific tissue or a specific disease can be conjugated to the microbubbles, enabling the microbubbles to accumulate selectively in areas of interest, such as diseased or abnormal tissues.

The term" microbubble" as used herein refers to an aggregate of molecules comprising a shell and a gas core. Microbubbles may differ in their shell makeup, gas core makeup, and whether or not they are targeted. The shell of the microbubble can be composed of lipids, phospholipids, surfactants, proteins, such as albumin, sugars such as galactose, and biocompatible synthetic polymers. A lipid monolayer shell is preferred.

The selection of the shell material determines how easily the microbubble is taken up by the immune system, hence how long the microbubble is stable within a patient's body. A more hydrophilic material tends to be taken up more easily, which reduces the microbubble residence time. This reduces the time available for imaging. The shell material also affects microbubble mechanical elasticity. The more elastic the material, the more acoustic energy it can withstand before bursting.

The microbubble shell may be covered with a substrate layer. Alternatively, the substrate may be part of the shell. PEG in addition to the substrate may be used to cover the shell and temporarily "hides" the microbubble from the immune system uptake, increasing the amount of circulation time. The substrates may be bond covalently to the material of the shell (e.g. using carbodiimide or maleimide as crosslinking agents) or are attached to the microbubbles by electrostatic interactions (such as protein binding, e.g. biotin-streptavidin coupling). The substrate may be the same as defined before.

The gas core of the microbubbles can be composed of air, or heavy gases like perfluorocarbon, or nitrogen. Heavy gases are less water-soluble so they are less likely to leak out from the microbubble leading to microbubble dissolution. As a result, microbubbles with heavy gas cores last longer. Regardless of the shell or gas core composition, the microbubble size can be fairly uniform. They have preferably a range of 0.5 - 4 micrometres in diameter. That makes them smaller than red blood cells, which ensures that they cause no stenosis, also not of microcirculation. Therefore, size of the microbubbles is an important property that need to be controlled. Microbubble size may be controlled according to methods known in the art. For example, microbubbles with different sizes may be sorted according to their sizes. There are several methods known and reported in the literature for microbubble size sorting (e.g. differential centrifugation, separation due to gravity, acoustic bubble sorting, pinched flow separation). In other embodiments, the microbubble size may be controlled during the synthesis. For example, methods have been reported for monodisperse microbubble synthesis based on microfluidic flow focusing devices. The size of the microbubbles may be analysed according to methods known in the art. For example, the size of microbubbles may be measured using electrophoretic light scattering, light microscopy or electro-impedance volumetric zone sensing.

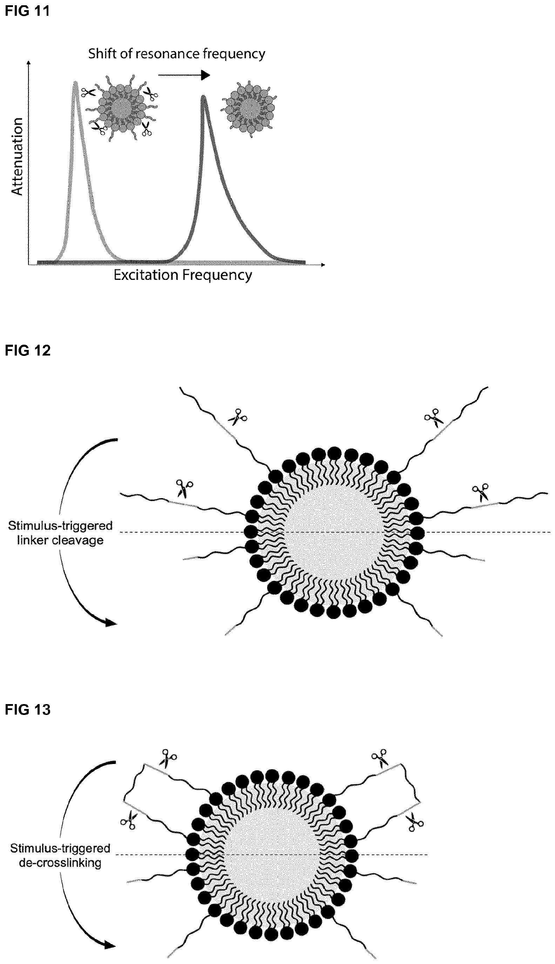

Microbubbles consisting of inert gas with lipid shells are known in prior art as ultrasound contrast agents. The present invention uses that their mechanical resonance varies with the mechanical properties of their shell. The microbubbles of the present invention show responsiveness caused by a modified chemistry or material of their shells. Cleavage of a substrate being part of the shell or linked to the shell results in an observable shift in resonance due to a change of the mechanical properties of the microbubble shell (Figure 11). The responsive element within a microbubble shell may be composed of a cleavable substrate functioning as a linker between the shell and auxiliary components.

The resonant frequency or characteristics of that resonance such as quality factor can be determined by different suitable ultrasonic method, such as a frequency sweep or the use of single arbitrary waveforms, and the detection of transmitted or reflected acoustic signals. In an ideal case, the method of detection could employ ultrasound equipment already present in clinics, however a purpose-specific detection device is also possible.

Preparation of the microbubbles according to the invention may be carried out according to methods known in the art, with appropriate modifications introducing the substrate. The shell of the microbubbles may comprise functional groups suitable for binding the substrate. Alternatively, the inventive (i.e. responsive) microbubbles may be prepared by functionalizing the bubble forming material. For some embodiments, the functionalization may be carried out after bubble formation. The functional groups may than be used to bind the substrate (or responsive element comprising the substrate).

The material forming the microbubble shell includes a substrate which may be part of a responsive element. The responsive element may comprise the substrate of an enzyme of interest and an inert component (a component not bond or amended by the enzyme). The microbubble shell forming material may contain one or more additional moieties. For example, the material forming the shell of the microbubble may consist of a bio-lipid with a polymer chain (e.g. PEG). This bio-lipid may be additionally functionalized at the end of the polymer chain. The structure and molecular weight of the attached polymer may be adjusted based on the needs of a particular application. It is preferred that the molecular weight of the PEG chain might vary between 1 and 5 kDa.

Possible mechanisms for introducing a substrate to the microbubbles in a way that cleavage alters the mechanical properties influencing the ultrasound signal include, but are not limited to:
The substrate links a component such as a polymer (release component) to the shell so that cleavage of the substrate releases this component, resulting in a change in mechanical properties of the shell including density, stiffness, or viscosity.

The substrate links a component such as a polymer to the shell so that cleavage of the substrate triggers a phase change of the component, resulting in a change in mechanical properties of the shell including density, stiffness, or viscosity. Alternatively, the enzyme activity triggers a phase change of the substrate that results in change of the ultrasound signal.

The substrate of the enzyme is a crosslinking element incorporating components that alters mechanical properties. Cleavage of the substrate eliminates cross linkage, resulting in changed ultrasound signals. An example may be cleavage and release of an auxiliary chain, as shown in Figure 12. An example of altered crosslinking is displayed in Figure 13, where auxiliary chains are de-crosslinked upon cleavage of the linker.

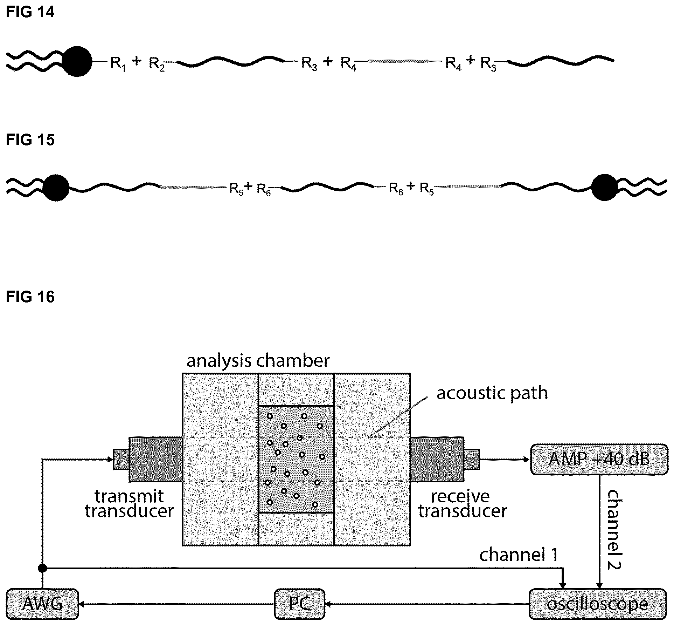

Possible arrangements of a cleavable linker and auxiliary components are shown in Figure 14 and Figure 15. The components can be conjugated via amine-reactive, carbodiimide, or sylfhydryl-reactive linker, or using click chemistry, among others. Examples of functional groups that can be deployed for linkage formation suitable for formation of microbubbles and magnetic particles according to the invention are listed in Table 1-6, and their pairings in Table 7-9.

**Table 1**

| Functionalization R₁ | | | |
|---|---|---|---|
| a | 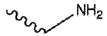 | b | 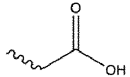 |
| c | 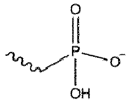 | | |

**Table 2**

| Functionalization R₂ | | | |
|---|---|---|---|
| a | 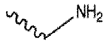 | b | 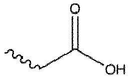 |
| c | 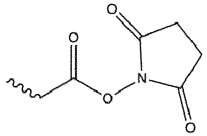 | | |

**Table 3**

| Functionalization R₃ | | | |
|---|---|---|---|
| a | 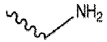 | b | 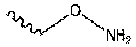 |
| c | 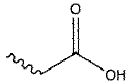 | d | 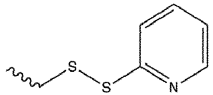 |
| e | 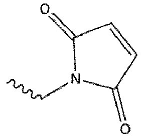 | f | 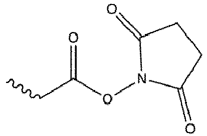 |
| g | 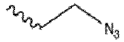 | h | 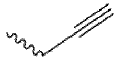 |
| i | 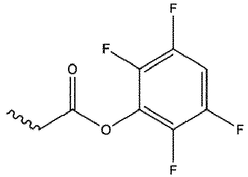 | j | 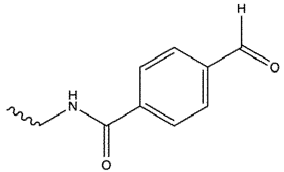 |

**Table 4**

| Functionalization R₄ | | | |
|---|---|---|---|
| a | 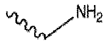 | b | 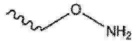 |
| c | 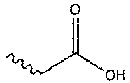 | d | 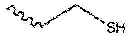 |
| e | 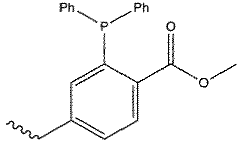 | f | 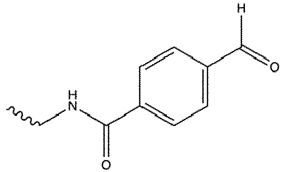 |
| g | 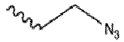 | h | 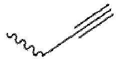 |

**Table 5**

| Functionalization R₅ | | | |
|---|---|---|---|
| a | 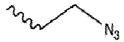 | b | 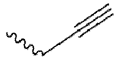 |

**Table 6**

| Functionalization R₆ | | | |
|---|---|---|---|
| a | 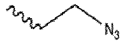 | b | 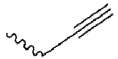 |
| c | 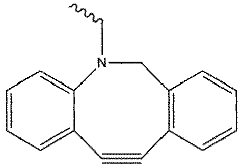 | | |

**Table 7**

| | R₂ | | |
|---|---|---|---|
| R₁ | | | |
| | a | b | c |
| a | | ★ | ★ |
| b | ★ | | |
| c | ★ | | |

**Table 8**

| | R₄ | | | | | | | |
|---|---|---|---|---|---|---|---|---|
| R₃ | | | | | | | | |
| | a | b | c | d | e | f | g | h |
| a | | | ★ | | | | | |
| b | | | | | | ★ | | |
| c | ★ | | | | | | | |
| d | | | | ★ | | | | |
| e | | | | ★ | | | | |
| f | ★ | | | | | | | |
| 9 | | | | | ★ | | | ★ |
| h | | | | | | | ★ | |
| i | ★ | | | | | | | |
| j | | ★ | | | | | | |

**Table 9**

| | R₆ | | |
|---|---|---|---|
| R₅ | | | |
| | a | b | c |
| a | | ★ | ★ |
| b | ★ | | |

Within the present invention, an optical read-out would be appreciated. For example, colour-based indication of the microbubble state should be visible on the ultrasound image. The ultrasound image indicating the microbubble state may be based on frequency spectrum analysis gained from signal post processing. Imaging frequencies for this application may be within 0.1 to 15 MHz or similar.

The ultrasound system converts the strong echogenicity into a contrast-enhanced image of the area of interest, revealing the location of the changed microbubbles.

Detection of microbubbles changed by the enzyme may then show that in the area of interest that particular enzyme is active, which can be indicative of a certain disease or disease state. Hence microbubbles according to the invention are suitable for use in a method of diagnosis in vivo of a disease associated with or caused by an altered activity of an enzyme of interest.

It is preferred that the disease associated with and/or caused by an altered (increased or decreased) enzyme activity is a disease associated with and/or caused by an altered protease activity. Diseases associated with and/or caused by an altered protease activity comprise arthritis, autoimmune diseases, bacterial or viral infections, cancer, impaired blood coagulation, cardiovascular diseases, lung diseases such as COPD or asthma, and metabolic diseases. The detection of enzyme activity using a method according to the present invention is further suitable to monitor the effect or success of an enzyme replacement therapy.

Advantages of the inventive method using ultrasound are: Ultrasound is very cost-efficient and widely available. Microbubbles can generate strong signals, therefore a low dosage may be used, being in the range of micrograms of microbubbles.

Advantages of the inventive method using magnetic nanoparticles are: The stability of the magnetic particle is higher, thus the residence time is longer. Free floating magnetic nanoparticles can easily be captured and carried out of the body.

Of course, the inventive magnetic nanoparticles as well as the inventive microbubbles may also be used to detect enzyme activity in samples derived from a patient's body or in other media such as waste. Therefore, the present invention refers also to an ex-vivo method for determining enzyme activity comprising the steps of:
a) providing a sample comprising an enzyme of interest
b) adding microbubbles of the invention or magnetic nanoparticle or respectively nanoaggregates of the invention to the sample, and
c) determining enzyme activity by measurement of altered ultrasound signal of the microbubbles of the invention or respectively measurement of altered magnetic field of the magnetic nanoparticle or nanoaggregates of the invention.

Still another aspect of the present invention is the determination of enzyme activity to test the effect of chemical compounds, medication, food and/or diet on diseases associated with and/or caused by an altered activity of the respective enzyme, in particular in situ such as within animal bodies, including human bodies. A preferred embodiment of the present invention refers to methods for detection of enzyme activity, wherein not only the specific enzyme substrate but further a candidate substance to be tested is applied. This candidate substance is preferably applied together or subsequently to the substrate. The aim of the methods for detection of enzyme activity, comprising further application of a candidate substance is to find candidate substances having at least one biological or pharmaceutical effect on the activity of the respective enzyme. Thus, candidate substances are examined to search for inhibitors or for activators. In general inhibitors of a specific enzyme, in particular a specific protease are promising candidates for drug development.

The candidate substance to be tested within a method of the invention for detection of enzyme activity may be any type of chemical molecule. The method of the present invention is suitable for screening large compound libraries for substances modulating enzyme activity.

In a preferred embodiment of the methods according to the invention the candidate substance can be:
i) a small molecule,
ii) an aptamer,
iii) a peptide, a protein, or a protein complex,
iv) or an antibody.

The term small molecule refers to a low molecular weight organic compound, which is by definition not a polymer. In the field of pharmacology, it is usually restricted to a molecule that also binds with high affinity to biopolymers such as proteins, nucleic acids or polysaccharides. Small molecules are broadly used as enzyme inhibitors and may be a nucleotide analog.

Aptamers are oligonucleic acid (DNA or RNA aptamers) or peptide molecules (peptide aptamers) that bind to a specific target molecule. Aptamers can be used for therapeutic purposes as macromolecular drugs. Aptamers can be created by selection from a large random sequence pool.

Further preferred embodiments are evident from the dependent patent claims. Features of the method claims may be combined with features of the product claims and vice versa.

The subject matter of the invention will be explained in more detail in the following text with reference to exemplary embodiments which are illustrated in the attached drawings, in which:
- Figure 1: illustrates the principle of the inventive method.
- Figure 2: further illustrates the principle of the inventive method, comprising determination of inductive detection using a pulsed field magnetometer.
- Figure 3: illustrates a magnetometer according to the invention, wherein the pulse coil, sense coil and compensation coil consist of electrically conductive spirals patterned in the layers of a printed circuit board or other planar geometry to which conductive features can be added with high resolution and geometric symmetry. The simplified schematic of the detection circuit shows that the sense and compensation coil are inductively coupled to the applied field with opposite polarity. A variable resistor is used as indicated to finely adjust the cancellation of the sense and compensation coils or aid in background subtraction and the signal is amplified for detection. Schematics of possible pulse circuits are also shown, with the one on the left based on a silicon controlled rectifier and the one on the right based on a gas discharge tube. Depending on the particular components and values used, different types of pulse forms are possible, several of which have been simulated and are shown at the bottom of the figure.

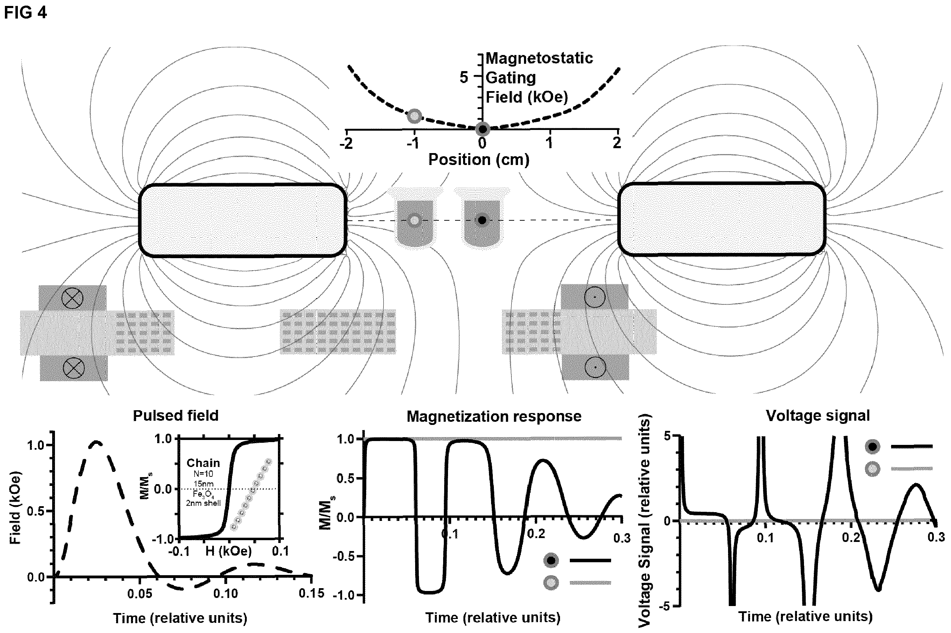

- Figure 4: shows an embodiment of the invention wherein a magnetostatic gating field is imposed by permanent magnets or electromagnets to produce spatially selective measurement of magnetic material. As an illustrative example, field lines are shown for a finite element simulation of two oppositely aligned bar magnets, and the expected field magnitude is plotted at positions along a dashed line. Two positions are considered, represented by two small vials at different positions on this line, however this strategy could equivalently be employed to isolate signal from a subvolume of a larger shape, such as a target of interest in a patient. Below, the plot on the left shows a simulated pulsed field versus time and a representative magnetization versus field curve for chains RIMAs produced from a 2D Monte Carlo simulation. At bottom center, the expected magnetization versus time is shown for the same RIMAs at these two positions. To the right, the resulting induced voltage signals show that a signal is only expected to arise from the RIMAs located at the point of vanishing magnitude of the field.
- Figure 5: illustrates variations on the arrangement of pulse, sense and compensation coils, some of which may be used to achieve increased penetration depth. In one variation, the sense and compensation coils are geometric mirror images and the pulsed field is symmetric, whereas in another the pulsed field is antisymmetric and the sense and compensation coils are identical. An antisymmetric pulse coil geometry may offer the advantage of reducing the inductance of the pulse coil, allowing for higher dH/dt values, and thus greater detection signal. Preferred pulse coil designs have few turns (as little as a single turn) to limit inductance and maximize dH/dt. Sense and compensation coils could consist of tens to hundreds of spiral turns embedded in a multilayer circuit board. While it is advantageous to have as many turns as possible in the sense coil to increase signal magnitude, the influence of parasitic capacitance limits the total number of allowable turns. Thus, the exact number of turns in the sense and compensation coils would be adapted for each setup. A concept for coupling to remote targets by moving the sense and compensation coils out of the plane of the pulse coil is shown at middle left. At middle right, a more general example is shown in which the compensation coil is reduced in diameter, but contains a different number of windings (40 times more than the sense coil in the example shown) and finely adjustable distance ensures cancellation of the voltage from the pulsed field. Such an arrangement could make remote measurement more feasible by permitting a large measurement coil that is as close to the target as the pulse coil, while reducing coupling of the compensation coil with the sample due to its smaller diameter.
- Figure 6: illustrates examples of voltage signals and their dependence of the conformation of assemblies from magnetic nanoparticles and pulse type. In 6A, at the top, two simulated pulsed fields are shown, an asymmetrically damped pulse and a monophasic pulse. At left, 2D Monte Carlo simulations of magnetization versus field curves for two different types of assemblies (chain and ring) are shown, along with the expected curves for their fully disassembled state. In the center of the figure, expected voltage signal versus time plots are shown for the different assemblies, paired with the two pulse types shown at the top. In 6B, alternative methods for detecting responses are sketched, including the observation of magnetic remanence or a measurable shift in complex susceptibility measured from a decaying oscillatory pulse.
- Figure 7: illustrates the basic concept of physical background subtraction possible within the present invention, respectively using the magnetometer of the invention. At top, the magnetometer is shown with no sample. Consistent with the concept of cancellation using the compensation coil and fine adjustment with the amplification circuit, no signal arises in this state. When a sample is introduced, its signal is formed from the superposition of two main components: diamagnetic contributions from the vial and solution, and the superparamagnetic signal of the magnetic assemblies. In the limit of small quantities of magnetic assemblies, the diamagnetic signal is expected to be considerably larger. By introducing a blank sample into the compensation coil, which does not contain magnetic assemblies, this comparatively large diamagnetic signal is physically subtracted, isolating the signal from the magnetic assemblies. Figure 8 shows the basic principle of responsive aggregates in the results of 2D Monte Carlo models of equilibrium magnetization versus field curves for chains and rings.

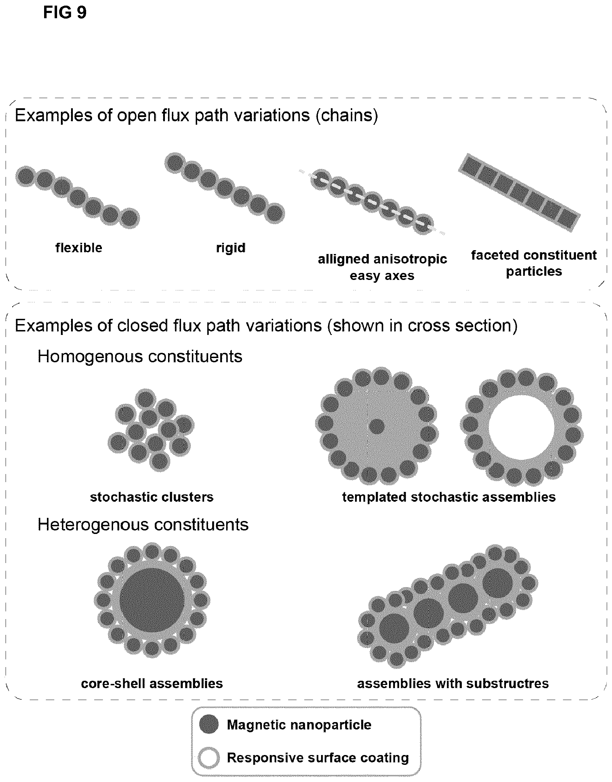

- Figure 9: shows a variety of possible structures for responsive magnetic nanoassemblies.
- Figure 10: A illustrates an example of chemical functionalization resulting in crosslinking of magnetic nanoparticles comprising a cleavable substrate suitable for the method of the invention.
B shows examples of functional groups suitable for bioconjugation of cleavable substrates to magnetic nanoparticles suitable for the method of the invention.
- Figure 11: illustrates a shift of resonance frequency after cleavage of a substrate from a microbubble.
- Figure 12: illustrates exemplarily how cleavage of a substrate can result in changed ultrasound signals. Shown is, that the cleavage causes release of an auxiliary chain.
- Figure 13: illustrates exemplarily how cleavage of a substrate can result in changed ultrasound signals. Shown is that the cleavage causes an altered crosslinking, where auxiliary chains are de-crosslinked upon cleavage of the linker.
- Figure 14: shows possible arrangements of a cleavable linker and auxiliary components as parts of a microbubble shell.
- Figure 15: shows possible arrangements of a cleavable linker and auxiliary components as parts of a microbubble shell.
- Figure 16: illustrates determination of microbubble resonance frequency using a custom-made attenuation measurement system.

The reference symbols used in the drawings, and their meanings, are listed in summary form in the list of reference symbols. In principle, identical parts are provided with the same reference symbols in the figures.

### Examples

All explanations of specific embodiments are included for illustrative purposes and not intended to be limiting. Reference is made to the accompanying figures in the application.

### Responsive magnetic nanoassemblies and an accompanying device that detects them via magnetic induction

### Example of synthesis and application:

### Synthesis of Iron and Cobalt Oleate

The synthesis of magnetic nanoparticles is well known to the art and many practical methods exist. The inventors used a previously described method based on metal-oleate decomposition, with slight modifications (Chen et al., DOI: 10.1021/acs.nanolett.5b04761, Nano Lett. 2016, 16, 1345-1351). 30 mmol of iron chloride hexahydrate are dissolved in 50 mL ultrapure (milliQ®) water and thoroughly degassed under vacuum. Atop this solution, the following are added sequentially: sodium oleate (90 mmol), hexane (100 mL), and finally ethanol (50 mL). The reaction is brought to reflux for 30 minutes under nitrogen and gentle stirring. Upon cooling, the hexane rich phase is isolated and washed 3 times with ultrapure (milliQ®) water (100 mL per wash) and finally kept at 135°C under atmosphere while stirring for 12 to 16 hours to remove volatiles. The final product is a dark brown, extremely viscous liquid. For cobalt doped magnetic nanoparticles, the only departure from the described procedure is a modification of the starting components. For a Co:Fe ratio of 1:11 (i.e. for CoₓFe₃₋ₓO₄, x = 0.25), 27.5 mmol of iron chloride hexahydrate, 2.5 mmol anhydrous cobalt chloride, and 87.5 mmol sodium oleate are combined.

### Synthesis of Magnetic Nanoparticles

In brief, 4.51g of the iron oleate or cobalt/iron oleate product described above is combined with 90% oleic acid and 15 mL of a co-solvent mixture of 1-octadecene and dibenzyl ether. The size of the particles is controlled by varying the amount of oleic acid, ratio of the co-solvents, and temperature ramp rate. Typically, a 1 to 2 dibenzyl ether to 1-octadecene ratio is used. The quantity of oleic acid added ranges from 1 mmol to 15 mmol. In each reaction, this mixture is degassed under stirring at 90°C for 30 minutes and an ultimate pressure of 0.1 mbar or less. The mixture is then heated rapidly to 200°C under nitrogen, typically ramped at a typical rate of 3.33°C/min to its reflux temperature, and maintained there for 30 minutes. Reflux temperature varies between approximately 290°C and 330°C depending on the composition of the mixture and on nitrogen pressure.

After synthesis, the particle-containing mixture is collected and washed from the flask using approximately 5mL hexane. The total solution is brought to 45mL with ethanol to help precipitate the particles and centrifuged at an RCF of 9000g for 10 minutes. After decanting, the pellet is redispersed under vortexing and sonication in 20 mL hexane with 1% oleic acid by volume, followed by the addition of 25mL of ethanol, further vortexing, and centrifugation. This process is repeated 4 to 5 times. Chloroform is used for the final resuspension of the pellet and this nanoparticle stock solution is stored at 4°C.

### Synthesis of PEG-PMAO and Phase Transfer

Synthesis of the amphiphilic polymer PEG-PMAO has been reported in many sources as an effective and biocompatible means to phase transfer nanoparticles (e.g. Yu et al., J. Am. Chem. Soc.2007129102871-2879). In a preferred method, 0.50g ofPMAO (1.4 mmol anhydride groups), 27.10 g of Jeffamine PEG diamine (14.3 mmol), and 0.87 g triethylamine (8.4 mmol) were combined in 175 mL dichloromethane under rapid stirring and allow the solution to react for 1 hour. The solution is then heated under nitrogen to its boiling point with the dichloromethane collected for disposal. Finally, the polymer rich residual solution is dried in vacuum overnight and the resulting powder is collected and stored under nitrogen.

For phase transfer, the typical method proceeds as follows. 1 mL of chloroform solution containing MNPs at a concentration of 1 mg of metal ion content per mL is prepared by diluting the stock solution with chloroform. 1.0 g of the solid product described above, containing both PEG-PMAO and excess unbound PEG is dissolved in 3 mL of chloroform. These solutions are bath sonicated separately for 15 minutes before mixing, and the combined mixture is sonicated for 30 minutes. The solution is then transferred to a large glass petri dish and dried overnight under vacuum. The polymer film containing evenly distributed nanoparticles is then dissolved in TRIS-acetate-EDTA buffer, sonicated for 15 minutes, and eluted through a 200 nm filter. Excess PEG and PEG-PMAO is washed away and the particles are transferred to their desired reaction buffers using MACS µ columns or other magnetic separation techniques and afterward sonicated for 15 minutes to ensure proper redispersion.

### Synthesis of Flux Closure IMAs and RIMAs

Upon phase transfer, the polymer surfaces of the particles contain free amine groups. To functionalize them with alkyne and azide moieties, the particles are reacted with propargyl-N-hydroxysuccinimidyl ester and azidoacetic acid NHS ester, respectively. For these reactions, the particles are transferred into 0.1 M sodium phosphate buffer with a pH value of 7.9. Approximately 1 mg of the desired NHS ester, enough to provide a substantial molar excess to the estimated available amine groups, is dissolved in a volume of DMSO equal to 10% of the total volume of the anticipated reaction mixture, which ranged from 200 µL to 1000 µL. Immediately after dissolution, the buffer containing the magnetic nanoparticles is combined and mixed thoroughly. After 4 hours at room temperature, dialysis or magnetic separation is used to remove excess click reagents and transfer the particles to 0.2M triethylammonium acetate buffer adjusted to a pH value of 7.0, and the particles are sonicated for 15 minutes to ensure proper redispersion.

To assemble the core shell structures, alkyne functionalized particles (e.g. 20 nm iron oxide) are combined with a 10-fold or greater excess of azide functionalized particles (e.g. 7.5 nm cobalt doped iron oxide). The following components are added to these solutions to initiate the click reaction: DMSO, a stock solution with 10 mM TBTA and copper (II) sulfate in 55% DMSO, and L-ascorbic acid freshly dissolved at 5 mM in water. The final reaction mixture contained 50% DMSO and overall concentrations of 0.1 M triethylammonium acetate buffer, 0.5 mM TBTA-Cu complex, and 0.5 mM ascorbic acid. Nitrogen is bubbled through the mixture and the vial containing the reaction mixture is flushed with nitrogen, sealed, and kept under agitation overnight. To separate the assemblies from excess unbound azide-functionalized nanoparticles, magnetic separation is employed, for instance using MACS µ columns with a water rinse step, a method that retains primarily the assemblies due to their larger magnetic moments, and elutes the smaller particles. After magnetic separation, the particles are sonicated for 15 minutes to ensure proper dispersion. To produce photo-responsive magnetic aggregates of this type, the NHS esters indicated above are substituted with photocleavable analogues containing the same end groups.

### Synthesis of Flux Reinforcement particles

In one method, particles phase transferred as described above are similarly coated in azide or alkyne groups and placed in click chemistry buffers. This time, they are combined with a crosslinker containing endgroups complimentary to their surface coatings such as PEG bis(azide), PEG bis(alkyne) or multi-arm variants of these. In a volume of 200 µL, and a concentration on the order of 100 µg to 1 mg per mL, the particles are placed in a uniform 0.8 T field. The concentration and time necessary for the formation of well controlled chains has to be determined on a case by case basis, but upon final combination of the TBTA-Cu catalyst, crosslinking is initiated and the structures are held in the assemblies they formed under the influence of the field.

Alternatively, flux reinforcement structures can be formed through the use of microfluidic device with permanent magnets incorporated to generate strong fields and gradients tangential to a 100 µm diameter PTFE tube. The crosslinking solution and particles are combined just before they enter the magnet, fed by syringe pumps at the rates ranging from 0.1 µL to 10s of µL per minute.

### Incorporation of Peptides into Flux Closure and Flux Reinforcement particles

In order to incorporate peptides, the above methodology requires modification. Rather than functionalizing the surface of the particles with click reactive moieties, the same procedure is adapted to 3-Mercaptopropanyl-N-hydroxysuccinimide ester by using a 0.1 M sodium phosphate buffer of the same pH, but also incorporating 10 mM EDTA and bubbling nitrogen through the solution beforehand. The reaction of this NHS ester provides thiol groups on the polymer surface of the nanoparticles. After magnetic separation, transfer to a 0.1 M sodium phosphate buffer of neutral pH with 10 mM EDTA, and sonication, 1 mg of a customized peptide is dissolved in a volume of DMSO sufficient for 10% of the total reaction volume and combined with the particles for 4 hours. The customized peptide includes a maleimide linker on one end that binds it selectively to the surface of the particles, as well as an alkyne containing unnatural amino acid at the opposite end. Synthesis of flux closure particles can then proceed as described above using click chemistry to affix particles directly functionalized with azide groups. Synthesis of the flux reinforcement particles proceeds as described above with the application of a uniform field and the use of a bis-azide, bis-alkyne, or multi arm crosslinker terminated with click groups.

### Use of magnetic particles to Detect Proteolytic Activity in ex vivo Samples

A trained medical professional is able to use established methods to collect a small liquid sample (less than 100 µL), e.g. of synovial fluid, which may or may not contain proteases of interest in their active form as a biomarker relevant to some disease or risk of disease. The *ex vivo* detection setup would be miniaturized so that the minimal dilution of the ex vivo sample would be required. An identical sample container, containing a physiological buffer, would be placed in the compensation coil. A general measuring procedure would begin by recording pulsed measurements using the sample without magnetic particles in the measurement coil and a blank in the compensation coil and either adjusting values of components in the amplification circuit or determining relevant values for digital background subtraction. The goal of this initial background calibration would be to eliminate background signal as fully as possible. A small quantity of particles would then be added to the physiological solution, in a concentration depending on the predetermined detection threshold of the setup, but likely ranging from 1 to 100 µg of material. The M vs H curve, or relevant property thereof, would then be inductively detected repeatedly for pulses spaced over minutes or hours, in effect measuring the time dependence of the change in the aggregation state of the particles. In each case, these curves could be compared against a control sample of particles (containing only physiological buffer) and a standard curve for known concentrations of the protease of interest to estimate the concentration of active protease. It would be possible to design a device in which the circuitry for pulse generation was shared such that these calibration curves could be run alongside measurement of the sample.

### Use of the magnetic particles in vivo

the magnetic particles with predetermined characteristics can be introduced into the body, either systemically in cases where accumulation in the site of interest is expected, or locally as in cases such as implant coatings or in situ analysis of synovial fluid. The necessary concentrations vary widely depending on the geometry of the detection apparatus, but are restricted to a total dose of mg scale or lower. For measurement, the site of interest should be placed in or near the pulse and detection coils. If necessary, background subtraction calibration could be performed on an analogous body part not expected to exhibit a signal from the used particles, such as a second hand or location of the body remote from the site of injection. With the pulse and detection coils in position over the site of interest, a magnetostatic selection field could additionally be applied to restrict inductively detectable signal to a point of vanishing magnitude. Repeated measurements over time indicate the aggregation state of the particles, and their rate of disassembly can be used to infer the activity of the protease of interest. It is worth noting that if properties of the M vs H curve such as saturation-normalized low field susceptibility are used, the relevant features are concentration independent. I.e. provided that a sufficient quantity of particles were being detected to provide a measurable signal, changes in their concentration through slow diffusion or other processes do not necessarily impact the ability of the device to measure a signal indicating their state of assembly.

### Responsive microbubbles with acoustic readout

### Example of synthesis and application:

### Synthesis of Microbubbles

The synthesis of lipid-based microbubbles is well known to the art and many practical methods exist. The inventors used a previously described method were primary and secondary lipids are added to a 25 mL borosilicate glass vial according to the ratios mentioned in the "Lipid Molar Ratios used for Microbubble Synthesis"-section and dissolved in chloroform. Chloroform is evaporated via a continuous stream of nitrogen to form a lipid thin film at the vial bottom. Remaining chloroform is then removed via desiccation overnight under house vacuum. The resulting lipid film is rehydrated using phosphate buffered saline (pH 7.4) to yield a final total lipid concentration of either 2 mg/mL (in case of sonication or shaking based synthesis) or 10 mg/mL (in case of flow focusing synthesis). The solution is stirred at 75 °C for at least 1 h and then bath sonicated for 20 min at RT. This process should yield a liposome solution with a mean liposome size of approximately 100 nm (measured by DLS). The formation of microbubbles is subsequently carried out with one of the following approaches:

### 1) Synthesis of Microbubbles by Sonication

The liposome solution can be heated above the phase transition temperature (approx.. 65 °C) and then probe sonicated (3 mm microtip, Branson Sonifier) for 10 s at 70 % amplitude under a continuous flow of perfluorobutane gas (PFB). After sonication, the white microbubble solution needs to be rapidly cooled below the phase transition temperature by placing the vial in an ice bath.

### 2) Synthesis of Microbubbles by Shaking

In this methods, a 2 mL aliquot of the liposome solution is transferred to a borosilicate vial (3.75 mL, 17 mm, septum lid) and heated above phase transition temperature (approx.. 65 °C). A 27-gauge needle, attached to a T-valve (connected to house vacuum and perfluorocarbon gas) needs to be inserted through the septum and the vial head space is exchanged five times with PFB. The vial is fixed in an amalgamator device (CapMix ESPE, 3M) and vigorously shaken for 30 s. After shaking, the white microbubble solution is rapidly cooled below the phase transition temperature by placing the vial in an ice bath.

### 3) Synthesis of Microbubbles by Flow Focusing

As reported previously by Segers et al. (DOI: 10.1021/acs.langmuir.6b00616 Langmuir 2016, 32, 3937-3944), a flow focusing microfluidic device can be utilized to synthesize monodisperse solutions of microbubbles. Here, the liposome solution is transferred to a 1 mL Hamilton syringe and connected to the liquid inlet of the mentioned microfluidic device using PTFE tubing. A syringe pump is used to control the liquid flow rate. The PFB gas pressure also needs to be controlled and connected to the gas inlet of the microfluidic chip by PTFE tubing. Depending on the desired size of microbubbles, liquid flow rate and gas pressure are adjusted based on empirical testing and verification for the given device.

### Lipid Molar Ratios used for Microbubble Synthesis

### a) Synthesis of Poly(acrylic acid) Microbubbles

Lipid A: DSPC
Lipid B: DSPE-PAA (synthesis see below)
Ratio of Lipid A:Lipid B = 170:1

### b) Synthesis of DSPE-mPEG2k and DSPE-mPEG5k Microbubbles

Lipid A: DSPC
Lipid B: DSPE-mPEG2k or DSPE-mPEG5k
Ratio of Lipid A:Lipid B = 99:1 for mushroom regime, 9:1 for brush regime, 3:1 for dense brush regime

### c) Synthesis of Microbubbles with Streptavidin Coating

Lipid A: DSPC
Lipid B: DSPE-PEG2k-biotin
Ratio of Lipid A:Lipid B = 9:1
After microbubble synthesis, streptavidin (excess to cover the microbubble surface, A_{streptavidin} = 19.63 nm²) is added to the microbubble solution and incubated for 1 h at RT to form a crystalline streptavidin protein shell.

### d) Synthesis of Microbubbles with Interconnected 4-arm PEG DSPE

Lipid A: DSPC
Lipid B: 4-arm-PEG-DSPE 20kDa (4-Arm PEG-DSPE is a 4-Arm PEG
with each of the four PEG arms terminated with a DSPE). It was purchased
from Creative PEG Works.
Ratio of Lipid A:Lipid B = 36:1

### Synthesis of DSPE-PAA

Following earlier protocols by Nakatsuka et al. (DOI: 10.1002/adma.201102677, Adv. Mater. 2011, 23, 4908-4912), a 50 wt% aqueous solution of poly(acrylic acid) (PAA, 200 mg, MW 5000 g/mol) is mixed with dimethylsulfoxide (DMSO, 4 mL) in a 25 mL borosilicate glass vial with a PTFE lid equipped with a magnetic stirbar. If necessary, 1 M aqueous hydrochloric acid solution is titrated dropwise to enhance solubility of the polymer. N-hydroxysuccinimide (NHS, 46 mg, 0.40 mmol) and 1-ethyl-3-(3-dimethylaminopropyl)carbodiimide hydrochloride (EDC, 38 mg, 0.20 mmol) are added and dissolved by stirring the solution for 30 min at room temperature. In a separate vial, a 5 mM solution of 1,2-distearoyl-sn-glycero-3-phosphorylethanolamine (DSPE, 15 mg, 0.02 mmol) in chloroform (4 mL) is prepared and mixed with trimethylamine (TEA, 1 mL). The mixture needs to be incubated at 60 °C until it appears as a clear solution. The two solutions are then combined under stirring, heated to 60 °C and finally allowed to react for 1 h. After cooling to room temperature, the chloroform and the TEA are removed via a continuous stream of nitrogen and transferred to a dialysis tube (MWCO 1 kDa). Dialysis was preferably performed during 2 days against distilled water with 8 changes of water. The dialyte is then lyophilized to obtain a white powder.

### Crosslinking of Poly(acrylic acid) Emulsifier Microbubbles

In order to create a crosslinked network on the surface of the microbubble, and therefore turn of the microbubble resonance ("OFF" mode), post processing of the microbubble in the "ON" mode needs to be carried out. After poly(acrylic acid) microbubble synthesis, excess of 4-arm PEG-amine (2 kDa, Creative PEG Works) and EDC are added to the microbubble solution. The solution is then transferred via a syringe to a septum lid vial filled with PFB gas and equipped with a venting needle. The solution is then set aside to react at RT overnight. To separate the microbubbles from unreacted material, the solution is transferred to a syringe and the microbubble solution is washed five times with PBS by centrifugation at 300 g for 10 min.

### Incorporation of Peptides into Microbubble Shells to Produce responsive microbubbles

In order to achieve microbubbles that are functionally responsive to proteolytic activity, selectively cleavable peptides need to be incorporated into the shell of the microbubble. Therefore, the above described methodologies require modifications.

### a) Synthesis of a responsive 4-armPEG-peptide crosslinker

The selectively cleavable peptide needed for this synthesis has an unnatural amino acid with an alkyne group (at the C terminus). The responsive crosslinker can be synthesized for example by combining a 4-arm PEG-azide linker with the peptide via click chemistry. For this, the 4-arm PEG-azide is combined with an eight-fold molar excess of the peptide. The following components are added to initiate the click reaction: DMSO, a stock solution with 10 mM TBTA and copper (II) sulfate in 55% DMSO, and L-ascorbic acid freshly dissolved at 5 mM in water. The final reaction mixture contained 50% DMSO and overall concentrations of 0.1 M triethylammonium acetate buffer, 0.5 mM TBTA-Cu complex, and 0.5 mM ascorbic acid. Nitrogen is bubbled through the mixture and the vial containing the reaction mixture is flushed with nitrogen, sealed, and kept under agitation overnight.

To separate unreacted material from the responsive crosslinker, the solution is transferred to a dialysis tube (MWCO 3.5 kDa). Dialysis is performed during 2 days against distilled water with 4 changes of water. The dialyte is lyophilized to obtain a white powder. MALDI-TOF can be used to verify if the functionalization of all 4 arms was successful.

Finally, the formation of responsive, crosslinked microbubbles can be performed in the same way as described above for the crosslinking of non-responsive microbubbles.

### b) Synthesis of a responsive DSPE-PEG2k-peptide-PEG3k emulsifier component

The selectively cleavable peptide needed for this synthesis has an additional cysteine at the N-terminus and an unnatural amino acid with an alkyne group at the C-terminus. In the first step of this two-step functionalization reaction the peptide is attached to a DSPE-PEG2k via sulfhydryl-reactive crosslinker chemistry. For example, DSPE-PEG2k-MAL is reacted with the cysteine of the peptide at the N-terminus with a 3:2 molar ratio in DMSO. The reaction is let run under nitrogen at room temperature overnight. To separate unreacted components from the product, the reaction solution is transferred into a dialysis tube (MWCO 3.5 kDa). Dialysis is performed during two days with four changes of water. The dialyte is lyophilized to obtain a white powder. In the second step of this synthesis, DSPE-PEG2k-peptide is attached to a PEG3k-alkyne via click chemistry. For this, DSPE-PEG2k-peptide is combined with a twofold molar excess of PEG3k-azide. Click chemistry is then performed as described above in section a). Unreacted materials are separated using a dialysis tube (MWCO 6-8 kDa).

Finally, responsive microbubbles can be synthesized as described above, but containing the responsive emulsifier component instead of the non-responsive DSPE-PEG2k or DPES-PEG5k.

### c) Synthesis of responsive DSPE-PEG2k-peptide-biotin

The selectively cleavable peptide needed for this synthesis has an additional cysteine at the N-terminus and a biotin functionalization at the C-terminus. Similarly, as described in section b), DSPE-PEG2k-MAL is reacted with the cysteine of the peptide via sulfhydryl-reactive crosslinker chemistry with a 3:2 molar ratio in DMSO. The reaction is let run under nitrogen at room temperature overnight. To separate unreacted components from the product, the reaction solution is transferred into a dialysis tube (MWCO 3.5 kDa). Dialysis is performed during two days with four changes of water. The dialyte is lyophilized to obtain a white powder.

Finally, responsive microbubbles with a streptavidin coating could be synthesized as described above, but containing the responsive DSPE-PEG2k-peptide-biotin instead of the non-responsive DSPE-PEG2k-biotin.

### d) Synthesis of responsive interconnected 4-arm PEG-peptide-PEG-DSPE

The selectively cleavable peptide needed for this synthesis has an additional cysteine at the N-terminus and an unnatural amino acid with an alkyne group at the C-terminus The first step of this two-step synthesis is similar to the one described in section a). For example, a 4-arm PEG-azide crosslinker can be combined with the peptide via click chemistry as described above. To separate unreacted material from the product, the solution is transferred to a dialysis tube (MWCO 3.5 kDa). Dialysis is performed during 2 days against distilled water with four changes of water. The dialyte is lyophilized to obtain a white powder. It is recommended to use MALDI-TOF to verify whether the functionalization of all 4 arms was successful.

For the second step of the synthesis 4-armPEG-peptide is combined with DSPE-PEG2k-MAL via sulfhydryl-reactive crosslinker chemistry with a 1:8 molar ratio in DMSO. The reaction is let run under nitrogen at room temperature overnight. To separate unreacted components from the product, the reaction solution is transferred into a dialysis tube (MWCO 6-8 kDa). Dialysis is performed during two days with four changes of water. The dialyte is lyophilized to obtain a white powder.

Finally, responsive microbubbles with a responsive interconnected shell can be synthesized as described above, but containing the responsive 4-arm PEG-peptide-PEG-DSPE instead of the non-responsive 4-arm PEG-DSPE.

### Measurements and analysis of microbubble resonance spectra

After synthesis, microbubble resonance frequency can be determined by any method known in the art. For our invention, a custom-made attenuation measurement system is used. The set up consists of 250 mL glass chamber to which a transmitting and a receiving transducer are attached coaxially (Figure 16). The intensity of the received signal is collected between 0.1 and 15 MHz. Intensities recorded from a suspension of microbubbles are compared to reference measurements and according to the following equation it is possible to calculate the attenuation coefficient. The resonance frequency of the microbubble suspension is then equal to the driving frequency at which the attenuation coefficient is the highest.

### Use of responsive microbubbles to Detect Proteolytic Activity in ex vivo Samples

A general measuring procedure can begin by diluting a small sample of synovial fluid (less than 100 µL) in PBS and transferring the solution to the ultrasound analysis chamber. First the acoustic background response of the solution needs to be measured by sending half-gaussian tapered sinusoidal waves at frequencies ranging from 0.1 to 15 MHz and with a total length of 12 µs through the measuring chamber in the absence of the responsive microbubbles. Following, an appropriate amount of microbubbles is added to the chamber. Appropriate concentrations of microbubbles need to be balanced between sufficient sensitivity for a given sample and attenuation of the ultrasound signal with increasing microbubble concentration. The measurement is repeated with the same signal as used for the background measurement. By subtracting the received signal intensities of the solution in presence of the microbubbles from the background, the resonance frequency of the microbubbles is determined. The sample with the responsive microbubbles should be incubated for several minutes and while repeated measurements are taken. In case of proteolytic activity, a shift in resonance frequency of the microbubbles due to changes in mechanical properties of the microbubble shell will be observed. In principle, it is also possible to determine the rate or reaction and therefore the concentration of the protease present in the chamber.

### Use of responsive microbubbles in vivo

For example, for diagnostics of inflammatory diseases of the joints, the microbubbles can be introduced locally into the synovial capsule of the affected joint via an intraarticular injection. The necessary concentration of microbubbles depends on the joint under examination and the distance of the ultrasound probe to the joint. After injection, the microbubbles can be detected using a clinical ultrasound imaging device. Using signal post-processing the frequency response of the microbubbles can be analyzed and visually encoded for image construction. A change in resonance frequency is visually detectable via a shift in contrast or color in respective areas of the ultrasound image. In some cases, the changes in mechanical shell properties should be so drastic that, proteolytic activity could also be visualized with harmonic imaging. For example, microbubbles with a responsive streptavidin coating are expected to be stiff enough to completely turn off harmonic response. After cleavage of the streptavidin, the shell softens and therefore an onset of harmonic responses should be observable. Similar effects are expected from the PAA-emulsifier microbubbles.

## Claims

1. A method for detection of enzymatic activity *in vivo* comprising the following steps:
a) incorporation of substrates being selectively cleavable by the enzyme of interest, and
b) non-invasive read-out of enzymatic activity.

2. The method according to claim 2, wherein the enzyme of interest is a hydrolase, in particular a protease or a peptidase.

3. The method according to claim 1 or 2, wherein step b) comprises determination of enzyme activity (locally) within a human or animal body.

4. The method according to any previous claim, wherein the substrate is conjugated to a magnetic nanoparticle or to a microbubble and wherein the nanoparticle or the microbubble alter a physical or physicochemical feature in response of substrate conversion by the enzyme of interest.

5. A responsive magnetic nanoparticle comprising a substrate for an enzyme of interest and being suitable to form magnetic nanoassemblies wherein the magnetic nanoassemblies produce a detectable signal difference in response of substrate conversion by the enzyme of interest.

6. The responsive magnetic nanoparticles according to claim 5, wherein the detectable signal difference is caused by disassembly in response to the activity of the enzyme of interest.

7. The responsive magnetic nanoparticles according to claim 5 or 6 for use in a method of diagnosis *in vivo* of a disease associated with or caused by an altered activity of the enzyme of interest.

8. A responsive microbubble comprising a substrates for an enzyme of interest and wherein the microbubble alters its reflection of ultrasound in response of substrate conversion by the enzyme of interest.

9. The responsive microbubble according to claim 8 made of lipids, phospholipids, surfactants, proteins, biocompatible synthetic polymers, or combinations thereof and being coupled to substrates for an enzyme of interest.

10. The responsive microbubble according to claim 8 or 9 for use in a method of diagnosis in vivo of a disease associated with or caused by an altered activity of the enzyme of interest.

11. A device suitable for the detection of enzymatic activity in vivo being a pulsed field magnetometer including a pulse coil with a drive circuit that generates a changing amplitude magnetic field for short durations in the range between 1 ns to 10 µs.

12. The device according to claim 11, wherein the changing magnetic field changes its amplitude from a range of 0 T to 1T within a period of Ins to 10 µs.

13. The device according to claim 11 or 12, wherein the changing magnetic field induces a voltage in a set of detection coils, which are inductively coupled to a sample and can be designed to isolate the voltage signal arising from its changing magnetization.

14. The device according to anyone of claims 11 to 13, wherein the pulse coil consists of electrically conductive spirals resting on either side of a printed circuit board or of a planar geometry on which conductive features can be patterned with high resolution and geometric symmetry.

15. The device according to anyone of claims 11 to 13, wherein the detection coil is incorporated as traces on a printed circuit board surrounding holes dimensioned to incorporate samples.
